# FASCICULE DE BREVET EUROPEEN

(11) **EP 0 574 077 B1**
(45) Date de publication et mention de la délivrance du brevet: **19.03.1997**
(21) Numéro de dépôt: 93201579.5
(22) Date de dépôt: 02.06.1993
(51) Int. Cl.: C07C 17/00, C07C 19/08

(54) **Procédé pour la préparation du 1,1,1,2-tétrafluoroéthane**
Verfahren zur Herstellung von 1,1,1,2-Tetrafluorethan
Process for the preparation of 1,1,1,2-tetrafluoroethane

(30) Priorité: 09.06.1992 BE 9200533
(43) Date de publication de la demande: 15.12.1993
(73) Titulaire: SOLVAY (Société Anonyme), B-1050 Bruxelles (BE)
(72) Inventeur: Pennetreau, Pascal, B-1330 Rixensart (BE); Janssens, Francine, B-1800 Vilvoorde (BE); Braun, Max, W-3006 Burgwedel 2 (DE); Eicher, Johannes, W-3008 Garbsen 5 (DE); Hausmann, Eckhard, W-3008 Garbsen 2 (DE); Rudolph, Werner, W-3000 Hannover 71 (DE)
(74) Mandataire: Jacques, Philippe

(56) Documents cités:
- EP-A- 0 036 123
- EP-A- 0 451 746
- EP-A- 0 462 645
- US-A- 4 258 225

## Description

L'invention concerne un procédé pour la préparation du 1,1,1,2-tétrafluoroéthane (HFA-134a) par réaction du fluorure d'hydrogène avec un trihalogénoéthylène.

Le 1,1,1,2-tétrafluoroéthane est un composé de synthèse contenant des atomes de carbone, de fluor et d'hydrogène mais pas de chlore. A ce titre, il peut constituer un substitut des hydrocarbures chlorofluorés entièrement halogénés (CFC) suspectés d'avoir un effet néfaste sur la couche d'ozone. Il s'avère notamment particulièrement intéressant, seul ou en mélange, dans certaines applications en réfrigération et en conditionnement d'air.

Le brevet US 4,258,225 divulgue un procédé pour la préparation de chlorofluoroalcanes par réaction du fluorure d'hydrogène avec une oléfine chlorée en phase liquide ou en phase gazeuse, en présence de pentafluorure de tantale ou de niobium. Ce procédé peut également être utilisé pour préparer le HFA-134a au départ de trifluoroéthylène.

La demande de brevet WO 89/12614 divulgue un procédé analogue utilisant le pentachlorure ou le pentabromure de tantale comme catalyseur.

La demande de brevet WO 91/18853 divulgue un procédé pour la préparation d'alcanes fluorés par réaction du fluorure d'hydrogène avec un alcane ou un alcène halogéné comprenant au moins un atome de chlore ou de brome, en présence d'un agent déshydratant et d'un catalyseur choisi parmi les oxydes de niobium et de tantale.

Pour obtenir du 1,1,1,2-tétrafluoroéthane par hydrofluoration d'oléfines en phase liquide, on suggère dans le brevet EP 300724 d'utiliser comme oléfine de départ un trihalogénoéthylène dont au moins un des atomes d'halogène n'est pas le fluor.

La demande EP-A-0451746 divulgue un procédé de fabrication du HFA-134a par réaction du fluorure d'hydrogène avec du trifluoroéthylène en phase liquide, en présence d'un système catalytique comprenant un dérivé d'un acide sulfonique et un halogénure métallique choisi parmi les pentahalogénures de niobium, de tantale et de molybdène.

La présente invention a pour but de fournir un procédé sûr, de coût réduit et de conduite aisée, pour la préparation du 1,1,1,2-tétrafluoroéthane par réaction du fluorure d'hydrogène avec un trihalogénoéthylène en offrant un taux de transformation du trihalogénoéthylène et une sélectivité en 1,1,1,2-tétrafluoroéthane élevés.

L'invention concerne dès lors un procédé pour la préparation du 1,1,1,2-tétrafluoroéthane par réaction du fluorure d'hydrogène avec un trihalogénoéthylène selon lequel on choisit comme trihalogénoéthylène le trifluoroéthylène et on met en oeuvre la réaction en phase liquide en présence d'un catalyseur qui comprend un halogénure et/ou un oxyde d'un métal choisi parmi le titane et l'étain.

Par halogénure et/ou oxyde d'un métal choisi parmi le titane et l'étain, on entend désigner un halogénure ou un oxyde de titane, un halogénure ou un oxyde d'étain, un mélange de ces halogénures et/ou de ces oxydes, un halogénure mixte de titane et d'étain, un oxyde mixte de titane et d'étain ou un oxyhalogénure de titane et/ou d'étain.

Par halogénure, on entend désigner un ou plusieurs sels formés à partir d'un ou plusieurs halogènes.

De préférence le catalyseur comprend au moins 50 % en poids d'halogénure et/ou oxyde d'un métal choisi parmi le titane et l'étain. Plus préférentiellement encore le catalyseur est essentiellement constitué d'halogénure et/ou oxyde d'un métal choisi parmi le titane et l'étain.

Le catalyseur mis en oeuvre est d'une préparation simple, est économique et offre un taux de transformation du trifluoroéthylène et une sélectivité en 1,1,1,2-tétrafluoroéthane très intéressants.

On retient avantageusement comme halogénure d'un métal choisi parmi le titane et l'étain un chlorure ou un fluorure. La mise en oeuvre d'un chlorure est avantageuse en ce que ce composé est économique et facilement disponible. On choisit en particulier un tétrachlorure. La mise en oeuvre d'un fluorure a également fourni de bons résultats. On choisit en particulier un tétrafluorure.

On choisit de manière particulièrement préférée comme halogénure un chlorofluorure. Cette variante de l'invention présenterait la particularité avantageuse de limiter d'abord la formation de chlorure d'hydrogène par réaction incidente du catalyseur avec le fluorure d'hydrogène utilisé pour traiter le trifluoroéthylène et, ensuite, la réaction subséquente du chlorure d'hydrogène ainsi formé avec le trifluoroéthylène pour former du 1-chloro-1,1,2-trifluoroéthane (HFA-133b) indésirable. Cette explication ne lie cependant pas la demanderesse. Plus préférentiellement encore, on met en oeuvre un composé de formule TiCl₄₋ₓFₓ ou SnCl₄₋ₓFₓ, x étant compris entre 0 et 4. D'excellents résultats ont été obtenus lorsque x est au moins égal à 1.

On utilise de manière avantageuse un catalyseur obtenu à partir d'un halogénure autre qu'un fluorure que l'on soumet préalablement à une fluoration au moins partielle. Cette fluoration peut en particulier être réalisée au moyen de fluorure d'hydrogène.

Comme oxyde de titane ou d'étain, on retient avantageusement un dioxyde de titane ou d'étain.

Dans le cas d'un oxyhalogénure, celui-ci peut être obtenu par exemple par une halogénation préalable d'un oxyde. On peut en particulier soumettre préalablement un oxyde à une fluoration au moins partielle. Cette fluoration peut plus particulièrement être réalisée au moyen de fluorure d'hydrogène.

Le titane est préféré comme métal, notamment au vu de ses moindres incidences sur l'environnement.

Dans le procédé selon l'invention, le catalyseur peut être mis en oeuvre en des quantités variables. Il est généralement utilisé à raison d'au moins 0,001 mole de catalyseur par mole de trifluoroéthylène. De très bons résultats ont été obtenus en présence d'au moins environ 0,005 mole de catalyseur par mole de trifluoroéthylène. En principe, il n'y a pas de limite supérieure à la quantité de catalyseur mise en oeuvre. En pratique toutefois, on n'utilise le plus souvent pas plus de 5 moles de catalyseur par mole de trifluoroéthylène. De préférence, on ne dépasse pas environ 0,5 mole et plus préférentiellement encore on ne dépasse pas environ 0,1 mole de catalyseur par mole de trifluoroéthylène.

Dans le procédé selon l'invention, le fluorure d'hydrogène et le trifluoroéthylène peuvent être mis en oeuvre dans des rapports molaires variables. Généralement, on met en oeuvre au moins 0,1 mole de fluorure d'hydrogène par mole de trifluoroéthylène. De préférence, ce rapport est d'au moins environ 1. Le plus souvent, on ne dépasse pas 20 moles de fluorure d'hydrogène par mole de trifluoroéthylène, les valeurs n'excédant pas environ 10 étant spécialement recommandées.

Le procédé selon l'invention peut être réalisé dans une large gamme de températures. Généralement, cette température est d'au moins environ 30 °C. De préférence, elle est d'au moins environ 50 °C. Une température d'au moins environ 70 °C permet une réaction plus rapide. Le plus souvent, en fonction notamment de la pression admissible, cette température ne dépasse pas environ 150 °C, les températures inférieures ou égales à environ 110 °C étant spécialement recommandées.

La pression n'est pas critique par elle-même, tant qu'elle permet de mettre en oeuvre le procédé selon l'invention en phase liquide. Cette pression peut être la pression autogène, une pression supérieure générée par l'introduction d'un gaz inerte, tel que par exemple l'azote, ou une pression inférieure obtenue par dilution du mélange réactionnel avec un solvant organique inerte, tel que par exemple le 1,2-dichloroéthane.

Le procédé selon l'invention peut être mis en oeuvre de manière continue ou discontinue. Le temps de séjour des réactifs dans le réacteur est généralement d'au moins environ 5 minutes. De préférence, il est d'au moins environ 15 minutes. Le plus souvent, ce temps de séjour ne dépasse pas environ 5 heures, les valeurs inférieures ou égales à environ 2 heures étant spécialement recommandées.

Le procédé selon l'invention peut être mis en oeuvre dans tout type de réacteur ou d'appareillage permettant de réunir les conditions décrites et en particulier de résister à la pression et au fluorure d'hydrogène. On utilise souvent des réacteurs en acier, en acier inoxydable ou en alliages tels que ceux connus sous les marques MONEL, INCONEL ou HASTELLOY. On peut également utiliser des réacteurs revêtus d'un métal ou d'un alliage inerte, ou recouverts d'une résine inerte dans les conditions du procédé, par exemple une résine fluorée.

Les exemples 2 à 5 ci-après illustrent l'invention.

### Exemple 1 (non conforme à l'invention)

On a réalisé un vide de 4.10³ Pa dans un réacteur de 250 cm³ muni d'une agitation magnétique, d'une prise de température et d'un manomètre et intérieurement recouvert de PVDF.

On y a ensuite introduit 31 g (1,55 mole) de HF à la température ambiante provenant d'une bonbonne pressurisée sous 4.10⁵ Pa par de l'azote. Le réacteur a ensuite été chargé de 58,7 g (0,716 mole) de trifluoroéthylène, également à la température ambiante.

On a placé le réacteur dans un bain d'huile préchauffé afin de l'amener et de le maintenir à une température intérieure d'environ 90 °C.

Les échantillons ont été prélevés en phase liquide, évacués au travers d'un scrubber contenant de l'eau à 40 °C et analysés de leur contenu en composés organiques par chromatographie en phase gazeuse.

Le tableau 1 illustre les conditions opératoires et les résultats obtenus.

**TABLEAU 1**

| | t=0 (*) | t=160 min | t=220 min |
|---|---|---|---|
| Température intérieure [°C] | - | 89,9 | 89,4 |
| Température bain d'huile [°C] | - | 129 | 131 |
| Pression [10⁵Pa] | - | 18,5 | 13,1 |
| Composition [%] | | | |
| Trifluoroéthylène | - | 54,7 | 41,5 |
| 1,1,1,2-tétrafluoroéthane | - | 45,3 | 58,5 |

| | | | |
|---|---|---|---|
| (*) 10 minutes avant d'avoir atteint la température souhaitée dans le réacteur, soit 60 minutes après introduction du réacteur dans le bain d'huile (pour tenir compte de la conversion en cours de montée en température). | | | |

### Exemple 2

On a introduit, à température et pression ambiantes, 5,9 g (0,030 mole) de SnF₄ dans un réacteur de 250 cm³ muni d'une agitation magnétique, d'une prise de température et d'un manomètre.

On a réalisé dans le réacteur un vide de 4.10³ Pa. On y a ensuite introduit 24,7 g (1,23 mole) de HF à la température ambiante provenant d'une bonbonne pressurisée sous 4.10⁵ Pa par de l'azote. Le réacteur a ensuite été chargé de 62,7 g (0,764 mole) de trifluoroéthylène, également à la température ambiante.

On a placé le réacteur dans un bain d'huile préchauffé afin de l'amener et de le maintenir à une température intérieure d'environ 90 °C.

Les échantillons ont été prélevés en phase gazeuse, évacués au travers d'un scrubber contenant de l'eau à 40 °C et analysés de leur contenu en composés organiques par chromatographie en phase gazeuse.

Le tableau 2 illustre les conditions opératoires et les résultats obtenus.

**TABLEAU 2**

| | t=0 (*) | t=25 min | t=55 min |
|---|---|---|---|
| Température intérieure [°C] | - | 86,8 | 91,4 |
| Température bain d'huile [°C] | - | 143 | 143 |
| Pression [10⁵Pa] | - | 25 | 25 |
| Composition [%] | | | |
| Trifluoroéthylène | - | 3,0 | 1,5 |
| 1,1,1,2-tétrafluoroéthane | - | 96,9 | 98,4 |

| | | | |
|---|---|---|---|
| (*) 10 minutes avant d'avoir atteint la température souhaitée dans le réacteur, soit 5 minutes après introduction du réacteur dans le bain d'huile (pour tenir compte de la conversion en cours de montée en température). | | | |

### Exemple 3

### a) Préparation du catalyseur

On a introduit à température et pression ambiantes 8 g (0,031 mole) de SnCl₄ dans un réacteur de 250 cm³, muni d'une agitation magnétique, d'une prise de température et d'un manomètre.

On a réalisé dans l'autoclave un vide de 4.10³ Pa et on y a introduit 34,2 g (1,71 mole) de HF à température ambiante, provenant d'une bonbonne pressurisée sous 4.10⁵ Pa par de l'azote.

On a ensuite chauffé le réacteur dans un bain d'huile jusqu'à ce qu'il ait atteint une température intérieure de 90 °C, que l'on a maintenue durant 6 heures.

On a alors refroidi le réacteur à la température ambiante et on a éliminé une partie de l'excès de HF ainsi que le HCl formé jusqu'à atteindre la pression atmosphérique, au travers d'un scrubber contenant 400 cm³ d'eau à 25 °C.

Le contenu du scrubber en Cl⁻ était alors de 1,39 g, ce qui correspond à un échange Cl⁻/F⁻ de 31,6 % et donc à une composition catalytique de formule SnCl_{2,7}F_{1,3}.

### b) Hydrofluoration du trifluoroéthylène

Le réacteur contenant le catalyseur préparé ci-avant a été maintenu à la température ambiante et placé sous un vide de 4.10³ Pa, ce qui a permis d'éliminer pratiquement la totalité du HF excédentaire mis en oeuvre lors de la préparation du catalyseur. Il a ensuite été alimenté à raison de 37,1 g (1,85 mole) de HF à température ambiante, provenant d'une bonbonne pressurisée sous 4.10⁵ Pa par de l'azote. Le réacteur a ensuite été chargé de 44,9 g (0,547 mole) de trifluoroéthylène, également à la température ambiante.

On a placé le réacteur dans un bain d'huile préchauffé afin de l'amener et de le maintenir à une température intérieure d'environ 90 °C.

Les échantillons ont été prélevés en phase gazeuse, évacués au travers du scrubber contenant de l'eau à 40 °C et analysés de leur contenu en composés organiques par chromatographie en phase gazeuse.

Le tableau 3 illustre les conditions opératoires et les résultats obtenus.

**TABLEAU 3**

| | t=0 (*) | t=55 min |
|---|---|---|
| Température intérieure [°C] | 76,5 | 94,5 |
| Température bain d'huile [°C] | 119 | 144 |
| Pression [10⁵Pa] | 20,5 | 27 |
| Composition [%] | | |
| Trifluoroéthylène | - | 0,35 |
| 1,1,1,2-tétrafluoroéthane | - | 99,5 |

| | | |
|---|---|---|
| (*) 10 minutes avant d'avoir atteint la température souhaitée dans le réacteur, soit 30 minutes après introduction du réacteur dans le bain d'huile (pour tenir compte de la conversion en cours de montée en température). | | |

Les exemples 2 et 3 illustrent l'excellent taux de transformation du trifluoroéthylène et la sélectivité très élevée en 1,1,1,2-tétrafluoroéthane obtenus au moyen du procédé selon l'invention, en présence d'un catalyseur à base d'étain.

### Exemple 4

### a) Préparation du catalyseur

On a introduit à température et pression ambiantes 6,1 g (0,0322 mole) de TiCl₄ dans un réacteur de 250 cm³, muni d'une agitation magnétique, d'une prise de température et d'un manomètre.

On a réalisé dans le réacteur un vide de 4.10³ Pa et on y a introduit 31,1 g (1,55 mole) de HF à température ambiante, provenant d'une bonbonne pressurisée sous 4.10⁵ Pa par de l'azote.

Après 30 minutes à température ambiante, on a éliminé une partie de l'excès de HF ainsi que le HCl formé jusqu'à atteindre la pression atmosphérique, au travers d'un scrubber contenant 400 cm³ d'eau à 25 °C.

Le contenu du scrubber en Cl⁻ était alors de 4,41 g, ce qui correspond à un échange Cl⁻/F⁻ de 96,6 % et donc à une composition catalytique de formule TiCl_{0,1}F_{3,9}.

### b) Hydrofluoration du trifluoroéthylène

Le réacteur contenant le catalyseur préparé ci-avant a été maintenu à la température ambiante et placé sous un vide de 4.10³ Pa, ce qui a permis d'éliminer pratiquement la totalité du HF excédentaire mis en oeuvre lors de la préparation du catalyseur. Il a ensuite été alimenté à raison de 28,7 g (1,43 mole) de HF à température ambiante, provenant d'une bonbonne pressurisée sous 4.10⁵ Pa par de l'azote. Le réacteur a alors été chargé de 52,4 g (0,639 mole) de trifluoroéthylène, également à la température ambiante.

On a placé le réacteur dans un bain d'huile préchauffé afin de l'amener et de le maintenir à une température intérieure d'environ 90 °C.

Les échantillons ont été prélevés en phase liquide, évacués au travers du scrubber contenant de l'eau à 40 °C et analysés de leur contenu en composés organiques par chromatographie en phase gazeuse.

Le tableau 4 illustre les conditions opératoires et les résultats obtenus.

**TABLEAU 4**

| | t=0 (*) | t=10 min | t=40 min |
|---|---|---|---|
| Température intérieure [°C] | - | 90,8 | 86,9 |
| Température bain d'huile [°C] | - | 110 | 121 |
| Pression [10⁵Pa] | - | 26,5 | 22,8 |
| Composition [%] | | | |
| Trifluoroéthylène | - | 10,9 | 0,4 |
| 1,1,1,2-tétrafluoroéthane | - | 88,9 | 99,5 |

| | | | |
|---|---|---|---|
| (*) 10 minutes avant d'avoir atteint la température souhaitée dans le réacteur, soit 5 minutes après introduction du réacteur dans le bain d'huile (pour tenir compte de la conversion en cours de montée en température). | | | |

L'exemple 4 illustre la préparation plus facile d'un catalyseur à base de titane et la plus grande rapidité du procédé selon l'invention en présence de ce catalyseur.

### Exemple 5

### a) Préparation du catalyseur

On a introduit à température et pression ambiantes 6,1 g (0,032 mole) de TiCl₄ dans un réacteur de 250 cm³, muni d'une agitation magnétique, d'une prise de température et d'un manomètre.

On a réalisé dans le réacteur un vide de 4.10³ Pa et on y a introduit 26,2 g (1,31 mole) de HF à température ambiante, provenant d'une bonbonne pressurisée sous 4.10⁵ Pa par de l'azote.

Après 30 minutes à température ambiante, on a éliminé une partie de l'excès de HF ainsi que le HCl formé jusqu'à atteindre la pression atmosphérique, au travers d'un scrubber contenant 400 cm³ d'eau à 25 °C.

Le contenu du scrubber en Cl⁻ était alors de 4,35 g, ce qui correspond à un échange Cl⁻/F⁻ de 95,3 % et donc à une composition catalytique de formule SnCl_{0,2}F_{3,8}.

### b) Hydrofluoration du trifluoroéthylène

Le réacteur contenant le catalyseur préparé ci-avant a été maintenu à la température ambiante et placé sous un vide de 4.10³ Pa, ce qui a permis d'éliminer pratiquement la totalité du HF excédentaire mis en oeuvre lors de la préparation du catalyseur. Il a ensuite été alimenté à raison de 37,1 g (1,67 mole) de HF à température ambiante, provenant d'une bonbonne pressurisée sous 4.10⁵ Pa par de l'azote. Le réacteur a ensuite été chargé de 50 g (0,61 mole) de trifluoroéthylène, également à la température ambiante.

On a placé le réacteur dans un bain d'huile préchauffé afin de l'amener et de le maintenir à une température intérieure d'environ 50 °C.

Les échantillons ont été prélevés en phase gazeuse, évacués au travers du scrubber contenant de l'eau à 40 °C et analysés de leur contenu en composés organiques par chromatographie en phase gazeuse.

Le tableau 5 illustre les conditions opératoires et les résultats obtenus.

**TABLEAU 5**

| | t=0 (*) | t=40 min | t=70 min |
|---|---|---|---|
| Température intérieure [°C] | - | 47,6 | 50,4 |
| Température bain d'huile [°C] | - | 57 | 61 |
| Pression [10⁵Pa] | - | 10,5 | 10 |
| Composition [%] | | | |
| Trifluoroéthylène | - | 6,3 | 1,8 |
| 1,1,1,2-tétrafluoroéthane | - | 93,6 | 98,1 |

| | | | |
|---|---|---|---|
| (*) 10 minutes avant d'avoir atteint la température souhaitée dans le réacteur, soit 5 minutes après introduction du réacteur dans le bain d'huile (pour tenir compte de la conversion en cours de montée en température). | | | |

L'exemple 5 illustre la faisabilité du procédé selon l'invention à plus basse température, mais avec une transformation moins rapide.

## Revendications

1. Procédé pour la préparation du 1,1,1,2-tétrafluoroéthane par réaction du fluorure d'hydrogène avec du trifluoroéthylène en phase liquide et en présence d'un catalyseur, caractérisé en ce que le catalyseur comprend un halogénure et/ou un oxyde d'un métal choisi parmi le titane et l'étain.

2. Procédé selon la revendication 1, caractérisé en ce que l'on choisit comme halogénure un chlorure ou un fluorure.

3. Procédé selon la revendication 2, caractérisé en ce que l'on choisit comme chlorure ou fluorure un tétrachlorure ou un tétrafluorure.

4. Procédé selon la revendication 1, caractérisé en ce que l'on choisit comme halogénure un chlorofluorure.

5. Procédé selon la revendication 4, caractérisé en ce que le chlorofluorure est un composé de formule TiCl₄₋ₓFₓ ou SnCl₄₋ₓFₓ, x étant compris entre 0 et 4.

6. Procédé selon la revendication 1, caractérisé en ce que le catalyseur est obtenu à partir d'un halogénure autre qu'un fluorure que l'on soumet préalablement à une fluoration au moins partielle.

7. Procédé selon la revendication 6, caractérisé en ce que la fluoration préalable est réalisée au moyen de fluorure d'hydrogène.

8. Procédé selon la revendication 1, caractérisé en ce que l'on choisit comme oxyde un dioxyde.

9. Procédé selon la revendication 1, caractérisé en ce que le métal choisi est le titane.

10. Procédé selon la revendication 1, caractérisé en ce que le catalyseur est mis en oeuvre à raison de 0,001 à 5 moles de catalyseur par mole de trifluoroéthylène.

11. Procédé selon l'une quelconque des revendications précédentes, caractérisé en ce que le fluorure d'hydrogène est mis en oeuvre à raison de 0,1 à 20 moles de fluorure d'hydrogène par mole de trifluoroéthylène.

## Patentansprüche

1. Verfahren zur Herstellung von 1,1,1,2-Tetrafluorethan durch Umsetzung von Fluorwasserstoff mit Trifluorethylen in flüssiger Phase und in Gegenwart eines Katalysators, dadurch gekennzeichnet, daß der Katalysator ein Halogenid und/oder ein Oxid eines Metalls enthält, das ausgewählt wird aus Titan und Zinn.

2. Verfahren nach Anspruch 1, dadurch gekennzeichnet, daß als Halogenid ein Chlorid oder ein Fluorid gewählt wird.

3. Verfahren nach Anspruch 2, dadurch gekennzeichnet, daß als Chlorid oder Fluorid ein Tetrachlorid oder ein Tetrafluorid gewählt wird.

4. Verfahren nach Anspruch 1, dadurch gekennzeichnet, daß als Halogenid ein Chloridfluorid gewählt wird.

5. Verfahren nach Anspruch 4, dadurch gekennzeichnet, daß das Chloridfluorid eine Verbindung der Formel TiCl₄₋ₓFₓ oder SnCl₄₋ₓFₓ ist, wobei x zwischen 0 und 4 beträgt.

6. Verfahren nach Anspruch 1, dadurch gekennzeichnet, daß der Katalysator ausgehend von einem vom Fluorid verschiedenen Halogenid gewonnen wird, welches zuvor einer zumindest teilweisen Fluorierung unterzogen wird.

7. Verfahren nach Anspruch 6, dadurch gekennzeichnet, daß die vorhergehende Fluorierung mit Hilfe von Fluorwasserstoff durchgeführt wird.

8. Verfahren nach Anspruch 1, dadurch gekennzeichnet, daß als Oxid ein Dioxid gewählt wird.

9. Verfahren nach Anspruch 1, dadurch gekennzeichnet, daß das gewählte Metall Titan ist.

10. Verfahren nach Anspruch 1, dadurch gekennzeichnet, daß der Katalysator im Verhältnis von 0,001 bis 5 mol Katalysator pro mol Trifluorethylen eingesetzt wird.

11. Verfahren nach einem beliebigen der vorhergehenden Ansprüche, dadurch gekennzeichnet, daß der Fluorwasserstoff im Verhältnis von 0,1 bis 20 mol Fluorwasserstoff pro mol Trifluorethylen eingesetzt wird.

## Claims

1. Process for the preparation of 1,1,1,2-tetrafluoroethane by reaction of hydrogen fluoride with trifluoroethylene in the liquid phase and in the presence of a catalyst, characterised in that the catalyst comprises a halide and/or an oxide of a metal chosen from titanium and tin.

2. Process according to Claim 1, characterised in that a chloride or a fluoride is chosen as halide.

3. Process according to Claim 2, characterised in that a tetrachloride or a tetrafluoride is chosen as chloride or fluoride.

4. Process according to Claim 1, characterised in that a chlorofluoride is chosen as halide.

5. Process according to Claim 4, characterised in that the chlorofluoride is a compound of formula TiCl₄₋ₓFₓ or SnCl₄₋ₓFₓ, x being between 0 and 4.

6. Process according to Claim 1, characterised in that the catalyst is obtained starting from a halide other than a fluoride which is subjected beforehand to an at least partial fluorination.

7. Process according to Claim 6, characterised in that the prior fluorination is carried out by means of hydrogen fluoride. ..

8. Process according to Claim 1, characterised in that a dioxide is chosen as oxide.

9. Process according to Claim 1, characterised in that the metal chosen is titanium.

10. Process according to Claim 1, characterised in that the catalyst is used at a concentration from 0.001 to 5 mol of catalyst per mole of trifluoroethylene.

11. Process according to any one of the preceding claims, characterised in that the hydrogen fluoride is used at a concentration from 0.1 to 20 mol of hydrogen fluoride per mole of trifluoroethylene.
